(19) **Europäisches Patentamt**

**European Patent Office**

**Office européen des brevets**

(11) **EP 0 700 217 B1**

(12) **EUROPÄISCHE PATENTSCHRIFT**

(45) Veröffentlichungstag und Bekanntmachung des Hinweises auf die Patenterteilung:
**07.11.2001 Patentblatt 2001/45**

(51) Int Cl.7: **H04N 9/73**

(21) Anmeldenummer: **95112637.4**

(22) Anmeldetag: **11.08.1995**

(54) **Verfahren und Vorrichtung zur Korrektur des Weissabgleichs eines Videofarbbildsignals**

Method and apparatus for correcting the white balance of a colour video signal

Procédé et appareil pour la correction de l'équilibre du blanc d'un signal vidéo couleur

(84) Benannte Vertragsstaaten:
**DE FR GB IT**

(30) Priorität: **03.09.1994 DE 4431491**

(43) Veröffentlichungstag der Anmeldung:
**06.03.1996 Patentblatt 1996/10**

(73) Patentinhaber: **Richard Wolf GmbH**
**75438 Knittlingen (DE)**

(72) Erfinder:
• **Häfele, Ulrich, Dipl.-Ing.**
  **D-75438 Knittlingen (DE)**
• **Vögele, Michael**
  **D-75236 Kämpfelbach (DE)**
• **Heinrichs, Jean-Pierre, Dipl.-Phys.**
  **D-75015 Bretten (DE)**

(74) Vertreter: **Wilcken, Thomas, Dipl.-Ing. et al**
**Patentanwälte**
**Wilcken & Vollmann**
**Bei der Lohmühle 23**
**23554 Lübeck (DE)**

(56) Entgegenhaltungen:
**EP-A- 0 400 606       EP-A- 0 530 738**
**US-A- 5 111 281**

**Beschreibung**

Die Erfindung betrifft ein Verfahren und eine Vorrichtung zur Korrektur des Weißabgleichs eines Videofarbbildsignals gemäß den Oberbegriffen der Patentansprüche 1 und 10.

In der Vergangenheit war das primäre Einsatzgebiet der Endoskopie die Diagnostik. Bei der Diagnostik betrachtet der Arzt das zu untersuchende Hohlorgan mit bloßem Auge. Das Auge besitzt eine hohe Helligkeits- und Farbdynamik, was zur Folge hat, daß die erreichbare Abbildungsqualität bezüglich Auflösung und Farbdarstellung nahezu nur von dem verwendeten Endoskop abhängig ist.

In der Chirurgie werden jedoch andere Anforderungen an ein endoskopisches System gestellt als bei der Diagnostik. Bei einem chirurgischen Eingriff muß dem gesamten Operationsteam das Bild des Operationsfeldes zur Verfügung stehen, da mehrere Personen den chirurgischen Eingriff in Zusammenarbeit durchführen.

Es reicht deshalb nicht aus, das Operationsfeld mit bloßem Auge durch ein Endoskop zu betrachten. Deshalb wird am proximalen Ende des Endoskops eine Videokamera aufgesetzt, oder es kommt ein Videoendoskop mit distal angeordnetem CCD Sensor zum Einsatz, so daß das Operationsfeld auf einem Monitor dargestellt werden kann. Mit dem Einsatz von CCD Kameras ergeben sich aber auch einige Probleme, da ein CCD Sensor nicht die Dynamik, die Empfindlichkeit und die spektralen Eigenschaften des Auges besitzt. Dies ist besonders in bezug auf die Farbdarstellung von Bedeutung. Es gilt nach einer Möglichkeit zu suchen, die Farbdarstellung unter dem Gesichtspunkt der auftretenden Farbschwankungen in einem endoskopischen System zu optimieren. Die Optimierung sollte möglichst automatisch ablaufen, damit sich das Operationsteam auf die Operation konzentrieren kann und sich nicht mit der Technik beschäftigen muß.

Bei der endoskopischen Untersuchung eines Hohlorganes des Körpers wird das Endoskop in die zu untersuchende Körperhöhle bzw. das Hohlorgan eingeführt. Beleuchtungslicht gelangt über Lichtleitfasern im Endoskop in die Körperhöhle, wo es das Hohlorgan beleuchtet. Um eine unverfälschte Farbwiedergabe zu erreichen, darf das Beleuchtungslicht nicht durch das Hohlorgan spektral beeinflußt werden. Dies ist jedoch, abhängig von der Beschaffenheit des Hohlorgans, nicht der Fall. Ein Teil des Beleuchtungslichtes dringt z.B. in die im Hohlorgan befindliche Schleimhaut, welche wie ein Absorptionsfilter wirkt, ein. Dadurch wirkt die Schleimhaut ihrerseits als eine spektral schmalbandige Beleuchtungsquelle, da das eingedrungene Beleuchtungslicht gefiltert wieder an der Oberfläche der Schleimhaut abgestrahlt wird und das zu betrachtende Objekt seinerseits mit rotem Licht beleuchtet.

Je nach untersuchtem Hohlorgan unterscheidet sich dieses Lichtabsorptionsverhalten. So spielt das Lichtabsorptionsverhalten im Gelenkbereich keine Rolle, während es im Magen, wo stark durchblutete Schleimhäute vorzufinden sind, zu einer Farbverschiebung in Richtung Rot kommt. Die Stärke der Rotverschiebung hängt bei einem gegebenen Organ vom Betrachtungsabstand und dem Betrachtungs- und Ausleuchtwinkel des Endoskops ab. Je kürzer der Betrachtungsabstand ist bzw. je näher das Endoskop sich an der Schleimhaut befindet, desto stärker wird die Schleimhaut durchleuchtet, was zu einer stärkeren Rotverschiebung führt. Wird der Betrachtungs- bzw. Ausleuchtwinkel größer, so ist die Intensität des Beleuchtungslichtes im Randbereich größer. In diesem Fall wird dort die Schleimhaut stark durchleuchtet. Besonders stark tritt dieser Effekt in der Gastroskopie und Coloskopie auf, bei denen der Blick-bzw. Ausleuchtwinkel mindestens 120° beträgt. Dabei werden röhrenartige Hohlorgane (Speiseröhre, Darm) untersucht, bei denen sich die Schleimhaut sehr nahe am Instrument befindet. In Verbindung mit dem großen Blick- bzw. Ausleuchtwinkel kommt es dort zu einer starken Rotverschiebung. Diese anwendungsspezifische Farbverschiebung wird jedoch bei Industrievideokameras nicht berücksichtigt.

Das US-Patent 5,111,281 beschreibt eine Farbkorrekturvorrichtung für ein Videoendoskop, die Mittel zur Erfassung der Farbqualität eines Farbbildsignals und Mittel zur pixelweisen Durchführung einer dynamischen Farbkorrektur aufweist. Aufgrund der pixelweise erfolgenden Korrektur ist die bekannte Vorrichtung nicht in der Lage, zwischen punktuell auftretenden starken Farben, insbesondere Rot, und Farberhöhungen, die das ganze Bild betreffen, zu unterscheiden, was zur Folge hat, daß die bekannte Vorrichtung auch punktuell auftretende Farberhöhungen korrigiert und damit eine schlechte Farbdifferenzierung hat.

Im US-Patent 4,831,437 wird ein Videoendoskop beschrieben, das mit einer Vorrichtung zum Farbabgleich versehen ist. Mit dieser Vorrichtung können jedoch nur die statischen Farbschwankungen, wie sie durch Lichtleitkabel, Endoskop und Beleuchtungsquelle hervorgerufen werden, im endoskopischen System ausgeregelt werden, d.h. dynamische Farbschwankungen, wie sie bei einem Eingriff durch die Schleimhaut auftreten können, werden nicht kompensiert.

Ferner zeigt das US-Patent 4,831,437 eine spezielle Vorrichtung zur Durchführung eines automatischen Weißabgleichs, die den Farbfehler beseitigt, welcher durch das Umgebungslicht hervorgerufen wird. Auch werden spektrale Differenzen der in endoskopischen Systemen verwendeten Komponenten, wie Lichtleiter, Lichtquelle und Endoskop, ausgeglichen. Die Vorrichtung hat zum Weißabgleich eine ballonartig geformte, auf der Innenseite weiß beschichtete Abgleichhilfe, durch deren Öffnung das Endoskop eingeführt wird. Dadurch ist gewährleistet, daß sich das den Abgleich störende Umgebungslicht nicht mit dem Beleuchtungslicht mischt. So wird erreicht, daß ein sich vor dem Endoskop befindliches weißes Betrachtungsfeld auch weiß auf dem Monitor dargestellt wird. Wird mit dem Endoskop jedoch ein Hohlorgan nach erfolgtem Weißabgleich untersucht, so kommt es durch die Schleimhaut zu einer Farbverschiebung in Richtung Rot.

Es ist deshalb Aufgabe der Erfindung, ein Verfahren und eine Vorrichtung zur Korrektur des Weißabgleichs eines Videofarbbildsignals anzugeben, die eine maximale Farbdifferenzierung des Farbbildes sicherstellen und eine spezifische Rotverschiebung im Farbbildsignal kompensieren können.

Ein die obige Aufgabe lösendes Verfahren zur Korrektur des Weißabgleichs eines Videofarbbildsignals besteht aus einem Schritt a) zur Erfassung einer Farbqualität des Farbbildsignals und ist gekennzeichnet durch folgende weitere Schritte:

b) Ermitteln von Korrekturwerten zur Korrektur des nach dem Weißabgleich vorliegenden Farbbildsignals für jede einzelne Farbkomponente in Abhängigkeit von vorgegebenen Betriebsbedingungen;
c) Korrektur des dem Weißabgleich entsprechenden Farbbildsignals durch die in Schritt b) ermittelten Korrekturwerte abhängig von einem Verhältnis der in Schritt a) erfaßten Rotkomponente zum Helligkeitsanteil der erfaßten Farbqualität des Farbbildsignals.

Bevorzugt werden in einem Speicherschritt vorab mehrere den Betriebsbedingungen entsprechende Sätze von Korrekturwerten gespeichert, wobei in Schritt b) die diesen Sätzen entsprechenden Korrekturwerte aus einem Speichermedium ausgelesen werden.

Zur Optimierung der Farbdifferenzierung weist das erfindungsgemäße Verfahren außerdem einen Integrationsschritt d) zur Integration wenigstens eines Teils der Farbkomponenten des Videofarbbildsignals über eine Halbbildperiode desselben auf. Bevorzugt integriert der Integrationsschritt d) nur Rot-Farbkomponenten R und Grün-Farb-komponenten G, und der Korrekturschritt c) korrigiert das Farbbildsignal nach dem Weißabgleich abhängig von dem Verhältnis $R/(R + G)$.

Zweckmäßigerweise werden die im erwähnten Speicherschritt gespeicherten Sätze von Korrekturwerten bzw. mehrere vorab gespeicherte Korrekturwertfunktionen abhängig vom Verhältnis $R/(R + G)$ ausgelesen.

Dabei können die gespeicherten Sätze von Korrekturwerten bzw. Korrekturwertfunktionen linear oder nicht-linear von dem Verhältnis der Rotkomponente zum Helligkeitsanteil des Farbbildsignals abhängen.

Das erfindungsgemäße Verfahren kann insbesondere auch zur Korrektur des Weißabgleichs von Videofarbbildsignalen bei Videoendoskopen oder Endoskopkameras angewendet werden.

Die erfindungsgemäße Vorrichtung zur Korrektur des Weißabgleichs eines Videofarbbildsignals weist eine Erfassungseinrichtung zur Erfassung einer Farbqualität eines empfangenen Farbbildsignals auf und ist gekennzeichnet durch:

- Mittel zur Ermittlung von Korrekturwerten zur Korrektur des Weißabgleichs für jede einzelne Farbkomponente in Abhängigkeit von vorgegebenen Betriebsbedingungen und

- Korrekturglieder zur Korrektur der dem Weißabgleich entsprechenden Farbkomponenten des Farbbildsignals durch die ermittelten Korrekturwerte abhängig von einem Verhältnis der Rotkomponente zum Helligkeitsanteil entsprechend der durch die Erfassungseinrichtung erfaßten Farbqualität.

Die Erfassungseinrichtung hat Glieder zur Messung der Intensität der Rotkomponente R und der Grünkomponente G des Farbbildsignals und Integrations- und Halteglieder zur Integration und zum Halten des Intensitätswertes jeweils der Rotkomponente R und der Grünkomponente G über eine Halbbildperiode des empfangenen Farbbildsignals.

Die Korrekturvorrichtung weist eine arithmetische Einheit zur Berechnung eines approximierten Helligkeitssignals jeweils aus der von den Integrations- und Haltegliedern über eine Halbbildperiode integrierten und gehaltenen Rotkomponente R und der über eine Halbbildperiode integrierten und gespeicherten Grünkomponente G, zur Berechnung eines Quotienten $R/(R + G)$ aus einer momentan gemessenen Intensität der Rotkomponente und des Helligkeitssignals und zur Ermittlung des Korrekturwerts auf.

Die arithmetische Einheit wird z.B. durch einen Mikroprozessor gebildet. Letzterer kann als Speichermittel einen Schreib-Lesespeicher aufweisen.

Dieser Schreib-Lesespeicher speichert mehrere vorgegebene Sätze von Korrekturwerten abhängig von dem Quotienten $R/(R + G)$ und von den vorgegebenen Betriebsbedingung in Form von Look-Up Tabellen, wobei der Mikroprozessor den jeweiligen Korrekturwert auf der Grundlage des aus der gemessenen momentanen Rotkomponente R und des Helligkeitssignals $R + G$ berechneten Quotienten $R/(R + G)$ aus der der jeweiligen Betriebsbedingung entsprechenden Look-Up Tabelle ausliest.

Mit dem Eingang des Mikroprozessors ist eine Bedieneinheit funktionell verbunden zur Auswahl eines von mehreren in den Schreib-Lesespeicher gespeicherten Sätzen von Korrekturwerten oder einer von mehreren von dem Quotienten aus der momentanen Rotkomponente R und dem annäherungsweise berechneten Helligkeitssignal $R + G$ abhängigen Korrekturwertfunktion. Zur Erzeugung einer der jeweiligen Farbkomponente entsprechenden Gleichspannung ist der Mikroprozessor ausgangsseitig mit je einem der Farbkomponente zugeordneten Korrekturspannungsgenerator verbunden, der vom Mikroprozessor den jeweils ausgewählten Korrekturwert für die jeweilige Farbkomponente empfängt. Jeder Korrekturspannungsgenerator weist einen Digital-Analogwandler bzw. ein digitales Potentiometer auf, bzw. das der eine analoge Korrekturgleichspannung erzeugt.

Außerdem ist der die jeweilige Korrekturspannung abgebende Ausgang jedes Digital-Analogwandlers mit einem Eingang eines spannungsgesteuerten Verstärkers verbunden, von dem ein anderer Eingang mit einem jeweiligen Ausgang einer Weißabgleichvorrichtung verbunden ist, die ihrerseits funktionell mit der Bedieneinheit zur Durchführung eines automatischen Weißabgleichs gekoppelt ist. Die Ausgänge der drei spannungsgesteuerten Verstärker führen jeweils zu Eingängen einer Matrixschaltung zur Erzeugung von Komponentensignalen.

Weitere Merkmale und Vorteile der Erfindung werden nachfolgend anhand von in der Zeichnung dargestellten Ausführungsbeispielen beschrieben. Es zeigt:

Fig. 1 eine Korrekturvorrichtung als Blockschaltbild,

Fig. 2 das Blockschaltbild der in Fig. 1 dargestellten Erfassungs- und Korrektur-Einrichtung,

Fig. 3 in einem Speichermittel gespeicherte Korrekturwertfunktionen und

Fig. 4 eine Hilfsvorrichtung für den automatischen Weißabgleich.

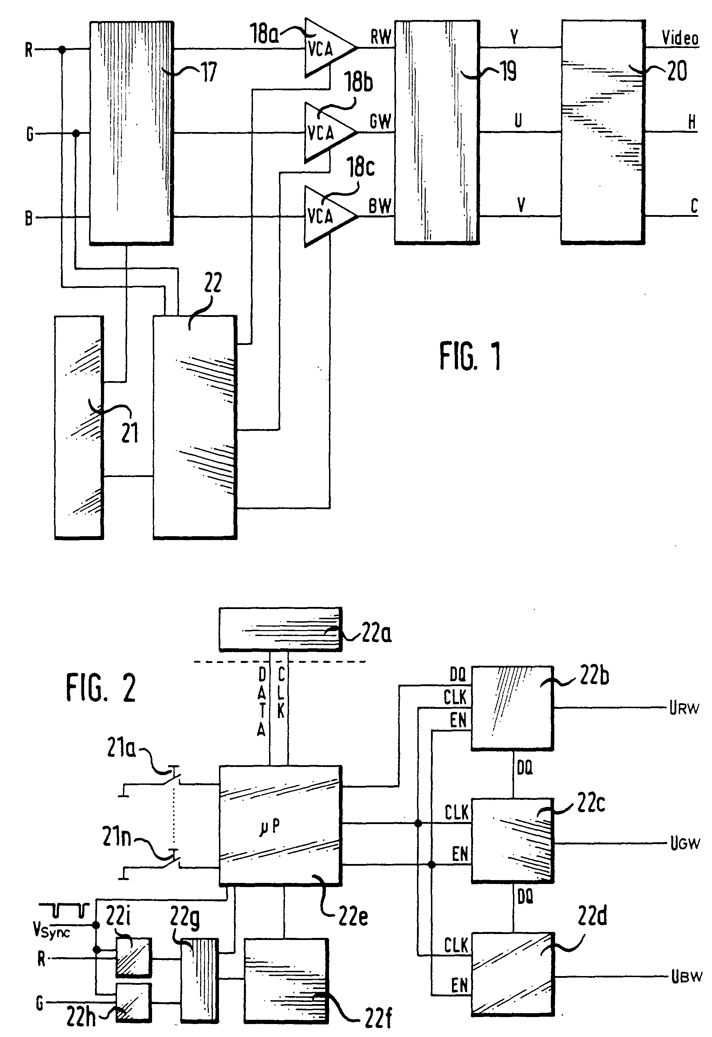

In Fig. 1 stehen am Eingang einer Einrichtung 17 zum automatischen Weißabgleich Videokameraausgangssignale R, G, und B an. Die Einrichtung 17 ist an sich bekannt und z.B in der EP-0530738A beschrieben. Sie wird von einer Bedieneinheit 21 aus gesteuert. Beim Betätigen einer Weißabgleichtaste wird ein automatischer Weißabgleich durchgeführt, wie er noch später näher erläutert wird.

Die abgeglichenen Signale, d.h. Farbkomponenten R, G, und B, gelangen vom Ausgang der Einrichtung 17 jeweils an einen Eingang von spannungsgesteuerten Verstärkern 18a, 18b und 18c, die nachstehend mit VCA bezeichnet werden. Die Steuereingänge sind jeweils mit einem Korrekturspannungsgenerator 22b, 22c, 22d verbunden.

Mit Hilfe der VCAs 18a, 18b, 18c und der Korrekturspannungsgeneratoren 22b, 22c und 22d kann der Weißabgleich anwendungsspezifisch korrigiert werden, um die durch die Schleimhaut im Hohlorgan des Körpers auftretende Farbverschiebung zu kompensieren. Die VCAs 18a, 18b und 18c werden von den Korrekturspannungsgeneratoren 22b, 22c, 22d jeweils mit einer Korrekturgleichspannung $U_{RW}$, $U_{GW}$, $U_{BW}$ angesteuert. Mit Hilfe dieser Spannungen werden die Verstärkungsgrade der VCAs eingestellt. Die so korrigierten Signale RW, GW, BW stehen am Eingang einer Matrix 19 an und werden dort in Komponentensignale Y, U, V umgewandelt. Ein der Matrix 19 nachgeschalteter Modulator 20 wandelt die Komponentensignale Y, U und V in das Helligkeitssignal H, das Chromasignal C und das Videosignal (Video) um. Die Matrix 19 und der Modulator 20 sind übliche integrierte, komplexe Bausteine.

Wie die Figuren 1 und 2 zeigen, erhält die Erfassungseinrichtung 22 eingangsseitig die Rot-Farbkomponente R und die Grün-Farbkomponente G des Farbbildsignals sowie einen Synchronisationsimpuls $V_{sync}$.

Erfindungsgemäß führt die Korrekturvorrichtung, wie sie in Fig. 1 und 2 dargestellt ist, eine dynamische Weißabgleichkorrektur durch. Die Erfassungseinrichtung mißt den Rotanteil und den Grünanteil des Farbbildes und bildet ein der Farbqualität entsprechendes Signal dadurch, daß der gemessene Rotanteil in bezug zu einem gemessenen Helligkeitsanteil des Bildes gesetzt wird. Übersteigt der Quotient aus dem Rotanteil und dem Helligkeitsanteil einen bestimmten Wert, so kann davon ausgegangen werden, daß ein erhöhter, in einem Bild unüblich großer Rotanteil, d.h. eine Farbverschiebung in Richtung Rot hervorgerufen durch die Schleimhaut des Hohlorgans im Körper vorliegt. Im Ausführungsbeispiel werden nur der Rotanteil und der Grünanteil der Farbkomponenten gemessen. Der Grünanteil des Bildes trägt am meisten zum Helligkeitssignal H des Farbbildes bei, der Blauanteil am wenigsten. Das Helligkeitssignal H setzt sich aus den Farbkomponenten R, G und B wie folgt zusammen:

$$H = 0,30R + 0,59G + 0,11B.$$

Diese Gleichung ergibt sich aus der Augenempfindlichkeitskurve für ein helladaptiertes Auge. Da somit der Blauanteil am wenigsten zum Helligkeitssignal beiträgt, werden nur der Rotanteil R und der Grünanteil G gemessen, und der Blauanteil B wird vernachlässigt.

Die Einrichtung 22 weist eine Arithmetikeinheit mit einem Mikroprozessor 22e auf. Dieser ist funktionell mit der Bedieneinheit 21 verbunden, die in Fig. 2 schematisch durch die Tasten 21a, ..... 21n dargestellt ist. An diesen Tasten können n verschiedene betriebs-bzw. anwendungsspezifische Einstellungen gewählt und zusätzlich die Farbeinstellung manuell verändert werden. Je nach gewählter Einstellung berechnet bzw. liest der Mikroprozessor 22e aus einer in einem Schreib-Lesespeicher 22a gespeicherten Tabelle der jeweiligen Anwendung bzw. Betriebsbedingung entsprechende Korrekturwerte für jede Farbkomponente aus, die an die als die Digital-Analogwandler arbeitenden Korrekturspannungsgeneratoren 22b, 22c und 22d ausgegeben werden.

Dieser Schreib-Lesespeicher 22a kann sich z. B. in einem Endoskop befinden und beinhaltet außer Abgleichdaten für das Videoendoskop auch eine Kennung, anhand der das Einsatzgebiet des Endoskops und somit auch die dafür optimale Weißabgleichfunktion bestimmt und automatisch auf eine der im Schreib-Lesespeicher 22a des Mikroprozessors 22e gespeicherte Korrekturwertfunktion geschaltet werden können. Im Schreib-Lesespeicher 22a können weiterhin auch Daten abgespeichert sein, die nicht die für das vorliegende Instrument optimierten Korrekturwerte betreffen.

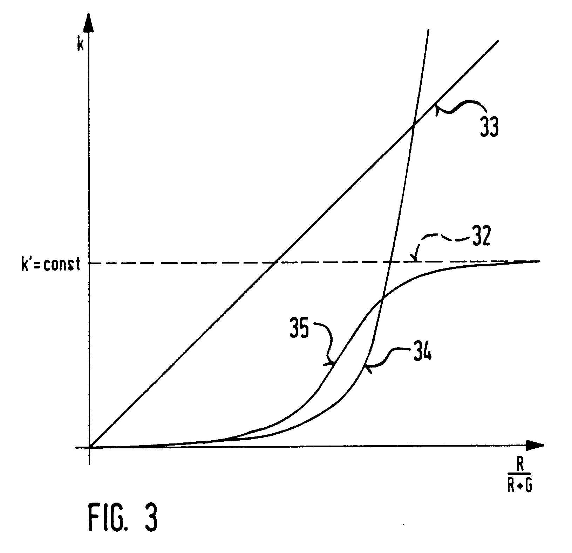

Fig. 3 zeigt mehrere mögliche und im Schreib-Lesespeicher 22a gespeicherte Funktionen des Korrekturwerts k in Abhängigkeit vom Verhältnis des Rotanteils zum Helligkeitsanteil des Farbbildsignals bzw. vom Quotienten $R/(R + G)$.

Zweckmäßigerweise ist jeder Farbe eine eigene Korrekturwertfunktion zugeordnet, obwohl Fig. 3 für jede Farbe nur jeweils eine Kurve zeigt. Die gestrichelt gezeichnete Gerade 32 entspricht einer statischen Weißabgleichkorrektur, d.h. der Korrekturwert k' ist konstant und hängt nicht vom Verhältnis $R/(R + G)$ ab. Der Einsatz einer solchen Weißabgleichkorrektur würde zur Folge haben, daß auch ein Bild mit geringem Rotanteil bereits eine Weißabgleichkorrektur erfahren und somit einen Farbstich außerhalb des Operationsfeldes oder auch bei großem Betrachtungsabstand dort, wo keine Rotverschiebung vorliegt, aufweisen würde. Die Gerade 33 zeigt eine lineare dynamische Funktion, wie sie als eine anwendungsspezifische Korrekturfunktion durch die erfindungsgemäße Korrekturvorrichtung verwendet werden kann. Hier wird mit steigendem Verhältnis Rotanteil/Helligkeitsanteil bzw. mit wachsendem Verhältnis $R/(R + G)$ des Bildes der Korrekturwert k linear vergrößert. Diese Korrekturwertfunktion ist besonders vorteilhaft, da bei einem größeren Betrachtungsabstand die Schleimhaut nicht so stark durchleuchtet wird und somit die Rotverschiebung geringer ist als bei kleinem Betrachtungsabstand, wo je nach Anwendung eine starke Rotverschiebung auftritt. Der Weißabgleich wird also nur so stark korrigiert, wie eine Verschiebung in Richtung Rot auftritt.

Dagegen weisen die Funktionen 34 und 35 ein ausgeprägtes nichtlineares Verhalten auf. Dies hat zur Folge, daß geringe Rotanteile, die z.B. nicht durch eine Rotverschiebung hervorgerufen sind, im Bild toleriert werden, also keine bzw. nur eine kleine Korrektur des Weißabgleichs hervorrufen. Stärkere Rotanteile, wie sie durch eine Rotverschiebung durch die Schleimhaut hervorgerufen werden, bewirken dagegen eine stärkere Weißabgleichkorrektur als bei linearem, dynamischen Verhalten. Während die Kurve 34 ab einem bestimmten Verhältnis $R/(R + G)$ stetig anwächst, hat die Kurve 35 eine asymptotische Annäherung an einen konstanten Korrekturwert, z.B. gemäß Fig. 3 an den der achsparallelen Geraden 32 entsprechenden Korrekturwert k'. Dies bewirkt, daß bei größeren Rotverschiebungen eine Weißabgleichkorrektur mit einem annähernd konstanten Korrekturwert k' erfolgt. Es soll jedoch ausdrücklich erwähnt werden, daß Fig. 3 nur Beispiele der Funktionen des Korrekturwerts k darstellt. Welche Korrekturwertfunktion letztendlich gespeichert bzw. ausgewählt wird, hängt von der Beschaffenheit des untersuchten Hohlorgans und von der darauf erfolgten Auswahl an der Bedieneinheit 21 bzw. ihren Tasten 21a,....21n durch die untersuchende Person ab. Es können also durchaus auch andere dynamische Verläufe des Korrekturwerts k im Schreib-Lesespeicher 22a gespeichert sein.

Der Mikroprozessor 22e erzeugt also die Korrekturwerte k für die Farbsignale RW, GW und BW in Abhängigkeit vom gemessenen Quotienten $R/(R + G)$ und der aus dem Schreib-Lesespeicher ausgewählten Korrekturwertfunktion (Fig. 3), während der Mikroprozessor 22e die jeweiligen Korrekturwerte auf der Basis von im Schreib-Lesespeicher gespeicherten, den Korrekturwertfunktionen entsprechenden Look-Up Tabellen erzeugt. Dies ist wesentlich schneller als eine Berechnung.

Für die Erfassung des Quotienten $R/(R + G)$ stehen die Videosignale R und G am Eingang von Integrations- und Haltegliedern 22h, 22i an und werden über ein Halbbild, d.h. über 20 ms integriert. Mit der negativen Flanke des Vertikalsynchronisierimpulses $V_{sync}$ wird die über ein Halbbild integrierte Spannung am Ausgang der Integrations- und Halteglieder 22h, 22i gespeichert. Diese Spannungen entsprechen dann der Intensität des Rot- und Grünanteils des Farbbildes. Die positive Flanke des Vertikalsynchronisierimpulses $V_{sync}$ setzt den Integrator der Integrations- und Halteglieder 22h, 22i zurück und löst am Mikroprozessor 22e eine Unterbrechung aus. Der Mikroprozessor 22e schaltet einen Multiplexer 22g auf den Ausgang des Integrations- und Halteglieds 22i. Somit steht die Spannung des Rot-Kanals am Eingang eines Analog-Digitalwandlers 22f an und wird dort digitalisiert. Dieser digitalisierte Wert wird im Mikroprozessor 22i zwischengespeichert. Mit Hilfe des neu erfaßten Rotanteils und des um ein Halbbild älteren Grünanteils wird ein Näherungswert des Helligkeitssignals gemäß der Summe R + G berechnet. Danach wird der Quotient des neuen Rotanteils gemäß $R/(R + G)$ berechnet.

Beim Ausführungsbeispiel liest der Mikroprozessor 22e auf der Basis des so berechneten Quotienten $R/(R + G)$ die an der Bedieneinheit 21 ausgewählten Weißabgleichkorrekturen aus der Look-Up Tabelle im Schreib-Lesespeicher 22a aus. Alternativ kann der Mikroprozessor 22e die dem Quotienten $R/(R + G)$ entsprechenden Korrekturwerte k für jede Farbe durch arithmetische Operation berechnen, was jedoch eine längere Rechenzeit als das Auslesen aus der Look-Up Tabelle braucht.

Mit dem nächsten Vertikalsynchronisierimpuls $V_{sync}$ wiederholt sich der beschriebene Einlese-Integrations- und Umsetzvorgang in derselben Art und Weise, nur daß nun der Grünkanal G eingelesen wird. Hierzu wird der Multiplexer 22g auf das Integrations-Halteglied 22h umgeschaltet. Mit Hilfe des neuen Grünanteils und des um ein Halbbild älteren Rotanteils wird wieder der Näherungswert für den Helligkeitsanteil, d.h. die Summe R + G berechnet. Nach dem Berechnen des Quotienten R/(R + G) werden anhand der gewählten Weißabgleichkorrekturfunktion die Korrekturwerte k für jede Farbe neu ermittelt, bzw. aus den im Schreib-Lesespeicher gespeicherten Look-Up Tabellen für die Korrekturwertfunktionen ausgelesen. Es genügt also, pro Halbbild die Intensität einer Farbkomponente R oder G zu messen und in den Mikroprozessor einzulesen, da sich innerhalb einer Halbbildperiode von 20 ms die über ein Halbbild integrierte Farbe nur um Bruchteile ändert. Es wäre auch denkbar, nach jeder Vollbildperiode den R-Anteil und den G-Anteil zu messen, also den R-Kanal und den G-Kanal in den Mikroprozessor 22e einzulesen und daraus den Korrekturwert k neu zu berechnen. Der Mikroprozessor 22e würde dadurch entlastet, da er nur noch halb so viele Operationen pro Zeiteinheit durchführen müßte. Dies hat jedoch zur Folge, daß die Korrekturspannungsgeneratoren 22b, 22c, 22d im 25 Hz Rythmus neu eingestellt werden und nicht mehr im 50 Hz Rythmus. Dies kann im extremen Fall zu einer Flimmerwirkung führen, da 25 Hz-Bilder in der Nähe der Wahrnehmbarkeitsgrenze des menschlichen Auges liegen.

Bevorzugt werden deshalb die Rot-Komponente und die Grün-Komponente jeweils um eine Halbbildperiode versetzt erfaßt und der ihrer Intensität entsprechende Digitalwert in den Mikroprozessor 22e eingelesen.

Die Korrekturspannungsgeneratoren 22b, 22c und 22d stellen Digital-Analogwandler bzw. digitale Potentiometer dar, sie erhalten vom Mikroprozessor 22e die den einzelnen Farbkomponenten entsprechenden digitalen Korrekturwerte k und wandeln diese in die Korrekturspannungen $U_{RW}$, $U_{GW}$ und $U_{BW}$ um, die den Steuereingängen der VCAs 18a, 18b und 18c zugeführt werden.

Die das Endoskop handhabende Untersuchungsperson kann zusätzlich zu der Auswahl der Korrekturwertfunktionen durch die Betätigung der Tasten 21a,...21n der Bedieneinheit 21 die anwendungsspezifischen Korrekturspannungen auch manuell auf Werte verändern, die nicht den Werten im Schreib-Lesespeicher 22a entsprechen. Diese manuelle Änderung kann durch Betätigung einer der Tasten 21a,....21n auch wieder rückgängig gemacht werden, so daß wieder die den vorgegebenen Funktionen entsprechenden Korrekturwerte k am Ausgang des Mikroprozessors 22e erzeugt werden.

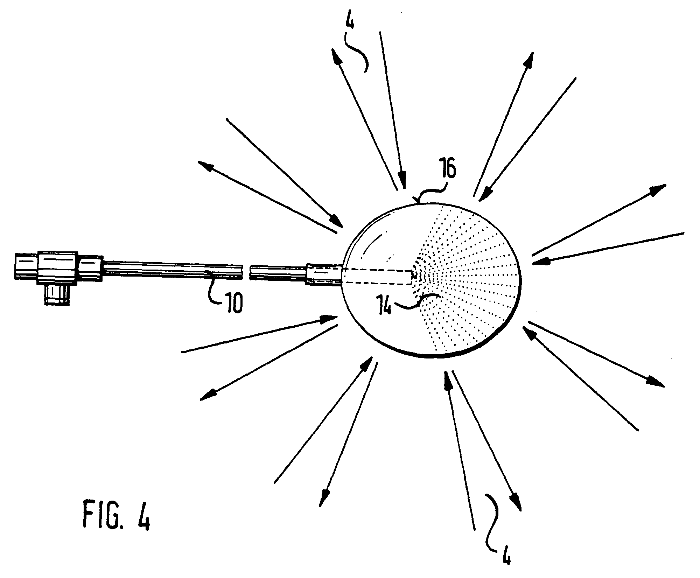

In Fig. 4 ist eine Hilfsvorrichtung für den Weißabgleich dargestellt, der mit der in Fig. 1 gezeigten Weißabgleichvorrichtung 17 erfolgt. Diese führt mit der Hilfsvorrichtung einen als EWC (Endo-White-Control) bezeichneten Weißabgleich durch. Ferner werden die spektralen Differenzen der in endoskopischen Systemen verwendeten Komponenten, wie Lichtleiter, Lichtquelle und Endoskop, ausgeglichen. Das Endoskop 10 wird durch eine Öffnung in eine ballonartig geformte, auf der Innenseite weiß beschichtete Abgleichhilfe 16 eingeführt. Somit ist gewährleistet, daß den Abgleich störendes Umgebungslicht 4 sich nicht mit dem endoskopischen Beleuchtungslicht mischen und zu einem falschen Ergebnis führen kann. Mit der Abgleichhilfe 16 und der Weißabgleichvorrichtung 17, wie sie aus der EP-0530738A bekannt ist, kann nun die Differenz der in einem endoskopischen System verwendeten Komponenten ausgeglichen werden. So wird erreicht, daß ein sich vor dem Endoskop befindliches weißes Betrachtungsfeld auch weiß auf dem Monitor dargestellt wird.

## Patentansprüche

1. Verfahren zur Korrektur des Weißabgleichs eines VideoFarbbildsignals mit einem Schritt a) zur Erfassung einer Farbqualität des Farbbildsignals, **gekennzeichnet durch** folgende weitere Schritte:

 b) Ermitteln von Korrekturwerten (k) zur Korrektur des nach dem Weißabgleich vorliegenden Farbbildsignals für jede einzelne Farbkomponente (R, G, B) in Abhängigkeit von vorgegebenen Betriebsbedingungen;
 c) Korrektur des dem Weißabgleich entsprechenden Farbbildsignals **durch** die in Schritt b) ermittelten Korrekturwerte (k) abhängig von einem Verhältnis der in Schritt a) erfaßten Rotkomponente (R) zum Helligkeitsanteil der erfaßten Farbqualität des Farbbildsignals.

2. Verfahren nach Anspruch 1, **dadurch gekennzeichnet, daß** in einem Speicherschritt vorab mehrere den Betriebsbedingungen entsprechende Sätze von Korrekturwerten (k) gespeichert werden und beim Schritt b) diese Sätze aus einem Speichermedium ausgelesen werden.

3. Verfahren nach Anspruch 1 oder 2, **dadurch gekennzeichnet, daß** Schritt a) außerdem einen Integrationsschritt d) zur Integration wenigstens eines Teils der Farbkomponenten (R, G, B) über eine Halbbildperiode des empfangenen Farbbildsignals einschließt.

4. Verfahren nach Anspruch 3, **dadurch gekennzeichnet, daß** der Integrationsschritt d) nur Rotund Grün-Farbkomponenten (R, G) integriert und der Korrekturschritt c) das nach dem Weißabgleich vorliegende Farbbildsignal abhängig von dem Verhältnis

$$R/(R + G)$$

korrigiert, wobei R die Rotkomponente und G die Grünkomponente ist.

5. Verfahren nach einem der Ansprüche 1 bis 4, **da-**

**durch gekennzeichnet, daß** die in Schritt b) ermittelten Korrekturwerte (k) linear von dem Verhältnis der Rotkomponente (R) zum Helligkeitsanteil des Farbbildsignals abhängen.

6. Verfahren nach einem der Ansprüche 1 bis 4, **dadurch gekennzeichnet, daß** die in Schritt b) ermittelten Korrekturwerte (k) nicht-linear von dem Verhältnis der Rotkomponente (R) zum Helligkeitsanteil des Farbbildsignals abhängen.

7. Verfahren nach einem oder mehreren der vorangehenden Ansprüche 2 bis 6, **dadurch gekennzeichnet, daß** die im Speicherschritt gespeicherten Sätze von Korrekturwerten (k) bzw. mehrere gespeicherte Korrekturwertfunktionen abhängig von dem Verhältnis R/(R + G) ausgelesen werden, wobei R die Rotkomponente und G die Grünkomponente ist.

8. Verfahren nach einem oder mehreren der Ansprüche 1 bis 7, **dadurch gekennzeichnet, daß** ein Auswahlschritt e) vorgesehen ist, um einen jeweils gewünschten Satz aus mehreren gespeicherten Sätzen von Korrekturwerten (k) bzw. jeweils eine gewünschte Korrekturwertfunktion aus mehreren gespeicherten Funktionen anwendungsspezifisch so auszuwählen, daß im Korrekturschritt c) eine anwendungsspezifische Korrektur durchgeführt wird.

9. Verfahren zur Korrektur des Weißabgleichs von Videofarbbildsignalen bei Videoendoskopen oder Endoskopkameras, gekennzeichnet durch die Anwendung des Verfahrens nach einem oder mehreren der Ansprüche 1 bis 8.

10. Vorrichtung zur Korrektur des Weißabgleichs eines Videofarbbildsignals mit einer Erfassungseinrichtung (22) zur Erfassung einer Farbqualität eines empfangenen Farbbildsignals, **gekennzeichnet durch**

   - Mittel (22e) zur Ermittlung von Korrekturwerten (k) zur Korrektur des Weißabgleichs für jede einzelne Farbkomponente (R, G, B) in Abhängigkeit von vorgegebenen Betriebsbedingungen und
   - Korrekturglieder (18a, 18b, 18c, 22a-22e) zur Korrektur der dem Weißabgleich entsprechenden Farbkomponenten des Farbbildsignals **durch** die ermittelten Korrekturwerte (k) in Abhängigkeit von einem Verhältnis der Rotkomponente zum Helligkeitsanteil entsprechend der **durch** die Erfassungseinrichtung (22) erfaßten Farbqualität.

11. Vorrichtung nach Anspruch 10, **dadurch gekennzeichnet, daß** die Erfassungseinrichtung (22) Glieder (22h, 22i) zur Messung der Intensität der Rotkomponente R und der Grünkomponente G des Farbbildsignals aufweist.

12. Vorrichtung nach Anspruch 11, **dadurch gekennzeichnet, daß** die Erfassungseinrichtung (22) Integrations- und Halteglieder (22h, 22i) aufweist zur Integration und zum Halten des Intensitätswertes jeweils der Rotkomponente R und der Grünkomponente G über eine Halbbildperiode des empfangenen Farbbildsignals.

13. Vorrichtung nach einem der Ansprüche 10 bis 12, **dadurch gekennzeichnet, daß** sie eine arithmetische Einheit (22e) zur Berechnung eines Helligkeitssignals (R + G) jeweils aus der von den Integrations- und Haltegliedern über eine Halbbildperiode integrierten und gehaltenen Rotkomponente R und der über eine Halbbildperiode integrierten und gehaltenen Grünkomponente G und zur Berechnung eines Quotienten R/(R+G) aus einer momentan gemessenen Intensität der Rotkomponente R und des berechneten Helligkeitssignals R + G und zur Ermittlung des Korrekturwertes aufweist.

14. Vorrichtung nach Anspruch 13, **dadurch gekennzeichnet, daß** die arithmetische Einheit (22e) ein Mikroprozessor ist.

15. Vorrichtung nach Anspruch 14, **dadurch gekennzeichnet, daß** der Mikroprozessor (22e) einen Schreib-Lesespeicher (22a) als Speichermittel aufweist.

16. Vorrichtung nach Anspruch 15, **dadurch gekennzeichnet, daß** der Schreib-Lesespeicher (22a) mehrere vorgegebene Sätze von Korrekturwerten (k) abhängig von dem Quotienten R/(R + G) und von den vorgegebenen Betriebsbedingungen in Form von Look-Up Tabellen speichert und daß der Mikroprozessor den jeweiligen Korrekturwert (k) auf der Grundlage des aus der gemessenen, momentanen Rotkomponente R und des Helligkeitssignals R + G berechneten Quotienten R/(R + G) aus der der jeweiligen Betriebsbedingung entsprechenden Look-Up Tabelle ausliest.

17. Vorrichtung nach einem der Ansprüche 14 bis 16, **gekennzeichnet durch** eine mit dem Eingang des Mikroprozessors (22e) funktionell verbundene Bedieneinheit (21, 21a,....21n) zur Auswahl eines von mehreren in dem Schreib-Lesespeicher gespeicherten Sätzen von Korrekturwerten (k) oder einer von mehreren von dem Quotienten aus der momentanen Rotkomponente R und dem berechneten Helligkeitssignal R + G abhängigen Korrekturwertfunktionen.

18. Vorrichtung nach einem oder mehreren der Ansprüche 14 bis 17, **dadurch gekennzeichnet, daß** der Mikroprozessor (22e) ausgangsseitig mit je einem der Farbkomponente zugeordneten Korrekturspannungsgenerator (22b, 22c, 22d) verbunden ist, der vom Mikroprozessor (22e) den jeweils ausgewählten Korrekturwert (k) für die jeweilige Farbkomponente empfängt, um daraus eine Korrekturgleichspannung ($U_{RW}$, $U_{GW}$, $U_{BW}$) zu erzeugen.

19. Vorrichtung nach Anspruch 18, **dadurch gekennzeichnet, daß** jeder Korrekturspannungsgenerator (22b, 22c, 22d) einen Digital-Analogwandler zur Erzeugung einer analogen Korrekturgleichspannung aufweist.

20. Vorrichtung nach Anspruch 19, **dadurch gekennzeichnet, daß** der die jeweilige Korrekturspannung ($U_{RW}$, $U_{GW}$, $U_{BW}$) abgebende Analogausgang jedes Digital-Analogwandlers mit einem Eingang eines spannungsgesteuerten Verstärkers (18a, 18b, 18c) verbunden ist, daß ein anderer Eingang jedes spannungsgesteuerten Verstärkers (18a, 18b, 18c) mit einem Ausgang einer Weißabgleichsvorrichtung (17) verbunden ist, die ihrerseits funktionell mit der Bedieneinheit (21, 21a,...21n) zur Durchführung eines automatischen Weißabgleichs gekoppelt ist, und daß die Ausgänge der drei Verstärker (18a, 18b, 18c) jeweils zu Eingängen einer Matrixschaltung (19) zur Erzeugung von Komponentensignalen (Y, U, V) geführt sind.

## Claims

1. Method for correcting the white balance of a video colour picture signal with a step a) for detecting a colour quality of the colour picture signal, **characterized by** following further steps:

   b) determination of correction values (k) for correcting the colour picture signal present after the white balance for each individual colour component (R, G, B) as a function of specified operating conditions;
   c) correction of the colour picture signal corresponding to the white balance by the correction values (k) determined in step b) as a function of a ratio of the red component (R) detected in step a) to the brightness proportion of the detected colour quality of the colour picture signal.

2. Method according to Claim 1, **characterized in that** in a storage step a plurality of sets of correction values (k) corresponding to the operating conditions are stored in advance, and these sets are read out from a storage medium in step b).

3. Method according to Claim 1 or 2, **characterized**

**in that** step a) also includes an integration step b) for the integration of at least some of the colour components (R, G, B) over a field period of the received colour picture signal.

4. Method according to Claim 3, **characterized in that** the integration step b) integrates only red and green colour components (R, G) and the correction step c) corrects the colour picture signal present after the white balance as a function of the ratio

$$R/(R + G)$$

where R is the red component and G the green component.

5. Method according to any one of Claims 1 to 4, **characterized in that** the correction values (k) determined in step b) are a linear function of the ratio of the red component (R) to the brightness proportion of the colour picture signal.

6. Method according to any one of Claims 1 to 4, **characterized in that** the correction values (k) determined in step b) are a nonlinear function of the ratio of the red component (R) to the brightness proportion of the colour picture signal.

7. Method according to one or more of the preceding Claims 2 to 6, **characterized in that** the sets of correction values (k) stored in the storage step and/or a plurality of stored correction-value functions are read out as a function of the ratio R/(R + G), where R is the red component and G the green component.

8. Method according to one or more of Claims 1 to 7, **characterized in that** a selection step b) is provided in order that one desired set from a plurality of stored sets of correction values (k) and/or one desired correction-value function from a plurality of stored functions may be selected in an application-specific way so that an application-specific correction is performed in the correction step c).

9. Method for correcting the white balance of video colour picture signals obtained by video endoscopes or endoscope cameras, **characterized by** use of the method according to one or more of Claims 1 to 8.

10. Apparatus for correcting the white balance of a video colour picture signal with a detection device (22) for detecting a colour quality of a received colour picture signal, **characterized by**

   - means (22e) for determining correction values (k) for correcting the white balance for each individual colour component (R, G, B) as a function of specified operating conditions and

- correction elements (18a, 18b, 18c, 22a-22e) for correcting the colour components of the colour picture signal corresponding to the white balance by the determined correction values (k) as a function of a ratio of the red component to the brightness proportion according to the colour quality detected by the detection device (22).

**11.** Apparatus according to Claim 10, **characterized in that** the detection device (22) has elements (22h, 22i) for measuring the intensity of the red component R and of the green component G of the colour picture signal.

**12.** Apparatus according to Claim 11, **characterized in that** the detection device (22) has integration and holding elements (22h, 22i) for integrating and holding the respective intensity values of the red component R and green component G over a field period of the received colour picture signal.

**13.** Apparatus according to any one of Claims 10 to 12, **characterized in that** it has an arithmetic unit (22e) for calculating a brightness signal (R + G) from the red component R integrated and held by the integration and holding elements over a field period and the green component G integrated and held over a field period and for calculating a quotient R/(R + G) by dividing an instantaneously measured intensity of the red component R by the calculated brightness signal R + G and for determining the correction value.

**14.** Apparatus according to Claim 13, **characterized in that** the arithmetic unit (22e) is a microprocessor.

**15.** Apparatus according to Claim 14, **characterized in that** the microprocessor (22e) has a read-write memory (22a) as storage medium.

**16.** Apparatus according to Claim 15, **characterized in that** the read-write memory (22a) stores a plurality of specified sets of correction values (k) as a function of the R/(R + G) quotient and of the specified operating conditions in the form of look-up tables and **in that** the microprocessor reads out each correction value (k) from the look-up table corresponding to the individual operating condition on the basis of the R/(R + G) quotient calculated from the measured instantaneous red component R and the brightness signal R + G.

**17.** Apparatus according to any one of Claims 14 to 16, **characterized by** a control unit (21, 21a, ... 21 n) functionally connected to the input of the microprocessor (22e), for selecting one of a plurality of sets of correction values (k) stored in the read-write memory or one of a plurality of correction-value functions which are functions of the quotient obtained by dividing the instantaneous red component R by the calculated brightness signal R + G.

**18.** Apparatus according to one or more of Claims 14 to 17, **characterized in that** the output side of the microprocessor (22e) is connected to correction voltage generators (22b, 22c, 22d) respectively assigned to each colour component, which each receive from the microprocessor (22e) the selected correction value (k) for the colour component concerned, in order to generate therefrom a correction DC voltage ($U_{RW}$, $U_{GW}$, $U_{BW}$).

**19.** Apparatus according to Claim 18, **characterized in that** each correction voltage generator (22b, 22c, 22d) has a digital/analogue converter to generate an analogue correction DC voltage.

**20.** Apparatus according to Claim 19, **characterized in that** the analogue output of each digital/analogue converter outputting the respective correction voltage ($U_{RW}$, $U_{GW}$, $U_{BW}$) is connected to an input of a voltage-controlled amplifier (18a, 18b, 18c), **in that** another input of each voltage-controlled amplifier (18a, 18b, 18c) is connected to an output of a white balance device (17) which is itself functionally coupled with the control unit (21, 21a, ... 21n) for carrying out an automatic white balance, and **in that** the outputs of the three amplifiers (18a, 18b, 18c) are respectively led to inputs of a matrix circuit (19) for generating component signals (Y, U, V).

**Revendications**

**1.** Procédé pour corriger l'équilibrage des blancs d'un signal d'image vidéo couleur, comprenant une étape a) de relevé d'une qualité de couleur du signal d'image couleur, **caractérisé par** les autres étapes suivantes :

b) détermination de valeurs de correction (k) destinées à la correction du signal d'image couleur présent après l'équilibrage des blancs pour chaque composante de couleur individuelle (R, V, B), en fonction de conditions de fonctionnement prescrites ;

c) correction du signal d'image couleur correspondant à l'équilibrage des blancs par les valeurs de correction (k) déterminées à l'étape b), en fonction d'un rapport de la composante rouge (R), relevée à l'étape a), à la fraction de luminance de la qualité de couleur relevée du signal d'image couleur.

2. Procédé selon la revendication 1, **caractérisé en ce que** lors d'une étape de mémorisation, on mémorise préalablement plusieurs groupes de valeurs de correction (k), qui correspondent aux conditions de fonctionnement, et ces groupes sont lus dans un moyen de mémorisation lors de l'étape b).

3. Procédé selon la revendication 1 ou 2, **caractérisé en ce que** l'étape a) englobe, en outre, une étape d'intégration d) pour l'intégration d'au moins une partie des composantes de couleur (R, V, B) sur une demi-période d'image du signal d'image couleur reçu.

4. Procédé selon la revendication 3, **caractérisé en ce que** l'étape d'intégration d) n'intègre que les composantes de couleur rouge et verte (R, V) et l'étape de correction c) réalise la correction du signal d'image couleur présent après l'équilibrage des blancs en fonction du rapport R/(R + V), R étant la composante rouge et V la composante verte.

5. Procédé selon l'une des revendications 1 à 4, **caractérisé en ce que** les valeurs de correction (k) déterminées à l'étape b) dépendent linéairement du rapport de la composante rouge (R) à la fraction de luminance du signal d'image couleur.

6. Procédé selon l'une des revendications 1 à 4, **caractérisé en ce que** les valeurs de correction (k) déterminées à l'étape b) dépendent non linéairement du rapport de la composante rouge (R) à la fraction de luminance du signal d'image couleur.

7. Procédé selon une ou plusieurs des revendications précédentes 2 à 6, **caractérisé en ce que** les groupes de valeurs de correction (k) mémorisés à l'étape de mémorisation ou plusieurs fonctions de valeurs de correction mémorisées, sont lus en fonction du rapport R/(R+V), R étant la composante rouge et V la composante verte.

8. Procédé selon une ou plusieurs des revendications 1 à 7, **caractérisé en ce qu'**il est prévu une étape de sélection e) en vue de sélectionner, de manière spécifique à l'application, un groupe souhaité respectif parmi plusieurs groupes de valeurs de correction (k) mémorisés ou une fonction souhaitée respective de valeurs de correction parmi plusieurs fonctions mémorisées, afin qu'une correction spécifique à l'application soit effectuée à l'étape de correction c).

9. Procédé de correction de l'équilibrage des blancs de signaux d'image vidéo couleur dans des endoscopes vidéo ou des caméras endoscopiques, **caractérisé par** l'utilisation du procédé selon une ou plusieurs des revendications 1 à 8.

10. Dispositif de correction de l'équilibrage des blancs d'un signal d'image vidéo couleur, comprenant un dispositif de relevé (22) destiné à relever une qualité de couleur d'un signal d'image couleur reçu, **caractérisé par**

- des moyens (22e) destinés à déterminer des valeurs de correction (k) en vue de la correction de l'équilibrage des blancs pour chaque composante de couleur (R, V, B) individuelle, en fonction de conditions de fonctionnement prescrites, et
- des organes de correction (18a, 18b, 18c, 22a-22e) pour la correction des composantes de couleur du signal d'image couleur correspondant à l'équilibrage des blancs, par les valeurs de correction (k) déterminées, en fonction d'un rapport de la composante rouge à la fraction de luminance conformément à la qualité de couleur relevée par le dispositif de relevé (22).

11. Dispositif selon la revendication 10, **caractérisé en ce que** le dispositif de relevé (22) comporte des organes (22h, 22i) destinés à mesurer l'intensité de la composante rouge R et de la composante verte V du signal d'image couleur.

12. Dispositif selon la revendication 11, **caractérisé en ce que** le dispositif de relevé (22) comporte des organes d'intégration et d'entretien (22h, 22i) pour l'intégration et pour l'entretien de la valeur d'intensité respectivement de la composante rouge R et de la composante verte V, sur une demi-période d'image du signal d'image couleur reçu.

13. Dispositif selon l'une des revendications 10 à 12, **caractérisé en ce qu'**il comporte une unité arithmétique (22e) destinée au calcul d'un signal de luminance (R + V), respectivement à partir de la composante rouge R intégrée et entretenue sur une demi-période d'image, par les organes d'intégration et d'entretien, et de la composante verte V intégrée et entretenue sur une demi-période d'image, et destinée au calcul d'un quotient R/(R+V) d'une intensité instantanée mesurée de la composante rouge R et du signal de luminance R+V calculé, et à la détermination de la valeur de correction.

14. Dispositif selon la revendication 13, **caractérisé en ce que** l'unité arithmétique (22e) est un microprocesseur.

**15.** Dispositif selon la revendication 14, **caractérisé en ce que** le microprocesseur (22e) comprend, en guise de moyen de mémorisation, une mémoire à lecture-écriture (22a).

**16.** Dispositif selon la revendication 15, **caractérisé en ce que** la mémoire à lecture-écriture (22a) mémorise plusieurs groupes prescrits de valeurs de correction (k) en fonction du quotient R/(R+V) et des conditions de fonctionnement prescrites, sous la forme de tables à consulter, et **en ce que** le microprocesseur lit la valeur de correction (k) considérée dans la table à consulter correspondant à la condition de fonctionnement considérée, sur la base du quotient R/(R+V) calculé à partir de la composante rouge R instantanée, mesurée, et du signal de luminance R+V.

**17.** Dispositif selon l'une des revendications 14 à 16, **caractérisé par** une unité de commande (21, 21a, ....21n) reliée fonctionnellement à l'entrée du microprocesseur (22e), pour la sélection d'un de plusieurs groupes de valeurs de correction (k) mémorisés dans la mémoire à lecture-écriture, ou d'une de plusieurs fonctions de valeurs de correction dépendant du quotient de la composante rouge R instantanée et du signal de luminance R+V calculé.

**18.** Dispositif selon une ou plusieurs des revendications 14 à 17, **caractérisé en ce que** le microprocesseur (22e) est relié, côté sortie, pour chaque composante de couleur, à un générateur de tension de correction (22b, 22c, 22d) associé, qui reçoit du microprocesseur (22e) la valeur de correction (k) respectivement sélectionnée pour la composante de couleur considérée, en vue d'engendrer à partir de celle-ci, une tension continue de correction ($U_{RW}$, $U_{VW}$, $U_{BW}$).

**19.** Dispositif selon la revendication 18, **caractérisé en ce que** chaque générateur de tension de correction (22b, 22c, 22d) comprend un convertisseur numérique / analogique en vue d'engendrer une tension continue de correction analogique.

**20.** Dispositif selon la revendication 19, **caractérisé en ce que** la sortie analogique de chaque convertisseur numérique / analogique, délivrant la tension de correction considérée ($U_{RW}$, $U_{VW}$, $U_{BW}$), est reliée à une entrée d'un amplificateur (18a, 18b, 18c) commandé en tension, **en ce qu'**une autre entrée de chaque amplificateur (18a, 18b, 18c) commandé en tension est reliée à une sortie d'un dispositif d'équilibrage des blancs (17), qui pour sa part est couplé de manière fonctionnelle à l'unité de commande (21, 21a,...21n) pour effectuer un équilibrage automatique des blancs, et **en ce que** les sorties des trois amplificateurs (18a, 18b, 18c) conduisent respectivement à des entrées d'un circuit de matrice (19) destiné à engendrer des signaux de composante (Y, U, V).

FIG. 1

FIG. 2

FIG. 3

FIG. 4